(19) Europäisches Patentamt
European Patent Office
Office européen des brevets

(11) Publication number: 0 098 138
A2

# (12) EUROPEAN PATENT APPLICATION

(21) Application number: 83303697.3

(22) Date of filing: 27.06.83

(51) Int. Cl.³: C 12 N 1/20
C 12 N 1/28, C 12 N 1/32
C 12 P 17/02, C 12 P 7/26
C 12 P 7/02, C 12 N 9/02
//C12R1/01, C12R1/04, C12R1/06,
C12R1/13, C12R1/15, C12R1/32,
C12R1/365, C12R1/38

(30) Priority: 28.06.82 US 392571
28.06.82 US 392611

(43) Date of publication of application:
11.01.84 Bulletin 84/2

(84) Designated Contracting States:
DE FR GB

(71) Applicant: Exxon Research and Engineering Company
P.O.Box 390 180 Park Avenue
Florham Park New Jersey 07932(US)

(72) Inventor: Hou, Ching-Tsang
14 Pendleton Place
Edison New Jersey(US)

(72) Inventor: Laskin, Allen I.
RD 3/Box 392T
Somerset New Jersey(US)

(72) Inventor: Patel, Ramesh Narsibhai
15 Mockingbird Road
Edison New Jersey(US)

(74) Representative: Field, Roger Norton et al,
ESSO Engineering (Europe) Ltd. Patents & Licences
Apex Tower High Street
New Malden Surrey KT3 4DJ(GB)

## (54) Microbial cells and the use thereof to produce oxidation products.

(57) Newly discovered and isolated microorganism strains and their natural mutants and genetically engineered derivatives are capable of growth under aerobic conditions in a culture medium containing a $C_2$-$C_{10}$ alkane or a $C_2$-$C_{10}$ alkyl radical donating compound as their major carbon and energy source. These microbial cells, which possess a high content of protein, can be utilized as feedstuffs or in oxidizing oxidizable organic substrates containing at least two carbon atoms, for example, converting alkanes and alicyclic and aromatic hydrocarbons to alcohols and/or ketones, alkenes, dienes, and vinyl aromatic compounds to 1,2-epoxides, and secondary alcohols to methyl ketones. Genetically engineered derivatives and enzyme preparations derived from these microbial cells may also be employed in the oxidative conversions.

EP 0 098 138 A2

The present invention relates to newly discovered and isolated microbial strains and their natural mutants and genetically engineered derivatives thereof which are capable of growth under aerobic conditions in a culture medium containing a $C_2$-$C_{10}$ alkyl compound such as, e.g., a $C_2$-$C_{10}$ alkane and a $C_2$-$C_{10}$ alcohol as their major carbon and energy source. The $C_2$-$C_{10}$ alkane-grown microbial cells or enzyme preparations derived therefrom are particularly useful in converting oxidizable organic substrates, e.g., alkanes, alkenes, dienes, alicyclic and aromatic hydrocarbons, and secondary alcohols, to the corresponding oxidized products.

Methane is not the only hydrocarbon substrate on which microorganisms can be grown. Depending on the particular bacterial strain employed, gaseous and liquid hydrocarbons have been known to be effective as growth substrates. The genera of microorganisms known to utilize hydrocarbons other than methane as carbon and energy source broadly include, e.g., Achromobacter, Acinetobacter, Pseudomonas, Nocardia, Bacillus, Mycobacterium, Corynebacterium, Brevibacterium, Candida, Arthrobacter, Streptomycetes, Flavobacterium, and various filamentous fungi. It is often difficult, however, to predict, for any given combination of microorganism strain and hydrocarbon growth substrate, the precise behavior of the strain in a culture medium.

U.S. Patent 3,344,037 discloses a method for enzymatically oxidizing an oxidized hydrocarbon such as an alcohol or aldehyde to an oxidation product with the same number of carbon atoms using a microorganism grown on a hydrocarbon corresponding substantially to the oxidizable hydrocarbon in number of carbon atoms. Preferably, the microorganism is chosen from the genera

of Achromobacter, Pseudomonas, Nocardia, Bacillus and Mycobacterium, most preferably Achromobacter grown on decane.

J. W. Foster, Ant. v. Leeu., J. Microbiol. & Ser., 28, 241 (1962) provides a review of organisms grown on hydrocarbons, and more recently, J. J. Perry, Microbiol. Rev., 43, 59 (1979) reviews studies of cooxidation of substrates using microorganisms grown on hydrocarbons. In co-oxidations, the non-growth substrates are oxidized when present as co-substrates in a growth medium containing the growth substrate. co-oxidation is thus not applicable to oxidations using resting cells.

A.S. Kester, PhD Diss., Univ. of Texas (1961) tested twenty-three microorganisms, representing the genera Nocardia, Brevibacterium, Pseudomonas, Alkaligenes or Achromobacter, Corynebacterium, Mycobacterium, Streptomyces and Fusarium for their capacity to grow on $C_1$-$C_{18}$ hydrocarbons, the capacities varying with the particular microorganism tested.

It has now been found that certain newly discovered and isolated microorganism strains and the natural mutants thereof and genetically engineered derivatives thereof are capable of growth under aerobic conditions in a culture medium containing a $C_2$-$C_{10}$ alkane, preferably a $C_2$-$C_4$ alkane, and most preferably propane, as the major carbon and energy source. The $C_2$-$C_{10}$ alkane-grown microbial cells, which possess a high content of protein and can be utilized as feedstuffs, are particularly useful in converting oxidizable organic substrates containing at least two carbon atoms to oxidation products, e.g., alkanes to the corresponding alcohols and/or methyl ketones, secondary alcohols to the corresponding methyl ketones, alicyclic and aromatic hydrocarbons to the corresponding hydrocarbyl alcohols and alkenes, dienes and vinyl aromatic compounds to the corresponding 1,2-epoxides.

It has also been found that these newly discovered and isolated microorganism strains may be aerobically grown on one of a plurality of $C_2$-$C_{10}$ alkyl radical donating compounds, such as, e.g., ethanol, propanol, butanol, ethylamine, propylamine, butylamine, ethyl formate, propyl formate, butyl formate, ethyl carbonate, propyl carbonate, butyl carbonate, etc., to produce microbial cells or enzyme preparations capable of aerobically converting secondary alcohols to the corresponding methyl ketones.

Specifically, the invention herein relates to isolated and biologically pure microbial cultures of microorganisms which utilize $C_2$-$C_{10}$ alkyl compounds, said cultures being selected from the group consisting of those having the following identifying characteristics:

| | | |
|---|---|---|
| Acinetobacter sp. | (CRL 67 P11) | NRRL B-11,313 |
| Actinomyces sp. | (CRL 66 P7) | NRRL 11,314 |
| Arthrobacter sp. | (CRL 60 P1) | NRRL B-11,315 |
| Arthrobacter sp. | (CRL 68 E1) | NRRL B-11,316 |
| Arthrobacter sp. | (CRL 70 B1) | NRRL B-11,317 |
| Brevibacterium sp. | (CRL 52 P3P) | NRRL B-11,318 |
| Brevibacterium sp. | (CRL 56 P6Y) | NRRL B-11,319 |
| Brevibacterium sp. | (CRL 61 P2) | NRRL B-11,320 |
| Corynebacterium sp. | (CRL 63 P4) | NRRL B-11,321 |
| Mycobacterium sp. | (CRL 51 P2Y) | NRRL B-11,322 |
| Mycobacterium sp. | (CRL 62 P3) | NRRL B-11,323 |
| Mycobacterium sp. | (CRL 69 E2) | NRRL B-11,324 |
| Nocardia sp. | (CRL 55 P5Y) | NRRL 11,325 |

| | | |
|---|---|---|
| *Nocardia* sp. | (CRL 57 P7Y) | NRRL 11,326 |
| *Nocardia* sp. | (CRL 64 P5) | NRRL 11,327 |
| *Pseudomonas* sp. | (CRL 53 P3Y) | NRRL B-11,329 |
| *Pseudomonas* sp. | (CRL 54 P4Y) | NRRL B-11,330 |
| *Pseudomonas* sp. | (CRL 58 P9Y) | NRRL B-11,331 |
| *Pseudomonas* sp. | (CRL 65 P6) | NRRL B-11,332 |
| *Pseudomonas* sp. | (CRL 71 B2) | NRRL B-11,333 |

and genetically engineered derivatives and natural mutants thereof, said cultures being characterized as capable of reproducing themselves and capable of producing dehydrogenase and/or monooxygenase enzyme activity in isolatable amounts when cultured under aerobic conditions in a liquid growth medium comprising assimilable sources of nitrogen and essential mineral salts in the presence of a $C_2$-$C_{10}$ alkyl compound as the major carbon and energy source.

In addition, the invention relates to a process for producing microbial cells which comprises culturing, under aerobic conditions, in a liquid growth medium containing assimilable sources of nitrogen and essential mineral salts in the presence of a $C_2$-$C_{10}$ alkyl compound, a microorganism strain chosen from the above-identified strains.

Furthermore, the invention is directed to a process for advancing an oxidizable organic substrate containing at least two carbon atoms to a state of oxidation greater than its original state, which comprises contacting said substrate, in a reaction medium under aerobic conditions, with cells of one of the above-identified microorganisms, a genetically engineered derivative or natural mutant thereof, or an enzyme preparation prepared from said cells or derivative, which microorganism, derivative, mutant or preparation exhibits oxygenase and/or hydrogenase enzyme activity, until at least a portion of the corresponding

oxidation product is produced in isolatable amounts, wherein said microorganism has been aerobically cultivated in a nutrient medium containing a $C_2$-$C_{10}$ alkyl compound.

The term "microorganism" is used herein in its broadest sense to include all of the microorganism strains identified below, which are generally bacterial strains.

The expression "genetically engineered derivatives of a microorganism" is used herein in the sense recognized by those skilled in the art, and includes artificial mutants and recombinant DNA-produced microorganisms.

The term "enzyme preparation" is used to refer to any composition of matter that exhibits the desired oxygenase or dehydrogenase enzymatic activity. The term is used to refer, for example, to live whole cells, dried cells, cell-free particulate and soluble fractions, cell extracts, and refined and concentrated preparations derived from the cells. Enzyme preparations may be either in dry or liquid form. The term also includes the immobilized form of the enzyme, e.g., the whole cells of the $C_2$-$C_{10}$ alkyl compound-grown microorganisms or enzyme extracts which are immobilized or bound to an insoluble matrix by covalent chemical linkages, absorption and entrapment of the enzyme within a gel lattice having pores sufficiently large to allow the molecules of the substrate and the product to pass freely, but sufficiently small to retain the enzyme. The term "enzyme preparation" also includes enzymes retained within hollow fiber membranes, e.g., as disclosed by Rony, Biotechnology and Bioengineering (1971).

The term "particulate fraction" refers to the enzyme activity in the precipitated or sedimented material when the supernatant after centrifuging broken cells at 5,000 x g. for 15 minutes is centrifuged for 1 hour at 10,000 x g. or greater.

The term "soluble fraction" refers to the enzyme activity in the soluble extract which is the supernatant after centrifugation of the broken cells at 10,000-30,000 x g for 15-30 minutes or greater and after successively stronger centrifugations if necessary.

The term "$C_2$-$C_{10}$ alkyl compound" refers to a $C_2$-$C_{10}$ alkane such as, e.g., ethane, propane, butane, isobutane, pentane, 2-methylbutane, hexane, octane, decane, etc., or a $C_2$-$C_{10}$ alkyl radical donating compound, which is a compound which will donate, e.g., ethyl, propyl, butyl, hexyl, decyl, etc. radicals, such as ethanol, propanol, 2-propanol, butanol, 2-butanol, hexanol, 2- or 3-hexanol, octanol, decanol, ethylamine, propylamine, butyl formate, ethyl carbonate, etc. Such alkyl radical donating compounds can also be characterized as growth substrates which are capable of inducing dehydrogenase enzyme activity in the microorganism strains described below. Preferably the $C_2$-$C_{10}$ alkyl compound herein is a $C_2$-$C_4$ alkyl compound, and more preferably $C_2$-$C_4$ n-alkanes, $C_2$-$C_4$ primary alcohols and $C_2$-$C_4$ alkylamines.

The term "advancing a substrate to a state of oxidation greater than its original state" refers to incorporation of oxygen or the removal of hydrogen in a $C_2$ or higher organic compound as substrate, such as where olefins are converted to 1,2-epoxides, alkanes are converted to alcohols and ketones, and secondary alcohols are converted to methyl ketones. Processes where the $C_2$-$C_{10}$ alkane-grown microbial cells or their

enzyme preparations are used to increase the oxidative state of an oxidizable organic substrate include converting alkenes (preferably $C_2$-$C_7$ alkenes, and most preferably $C_2$-$C_5$ alkenes), dienes and vinyl aromatic compounds such as ethylene, propylene, 1-butene, cis-but-2-ene, trans-but-2-ene, isobutene, 1-pentene, 2-methyl-1-butene, 1-hexene, 1-heptene, butadiene, isoprene, and styrene, to the corresponding 1,2-epoxides, converting alkanes (preferably $C_2$-$C_6$ alkanes) and alicyclic (i.e. cycloaliphatic) and aromatic hydrocarbons such as ethane, propane, butane, isobutane, pentane, 2,2-dimethylpropane, isopentane, neopentane, 2,2-dimethylbutane, 2,3-dimethylbutane, hexane, cyclopropane, cyclohexane, toluene, and benzene, to the corresponding alcohols, converting alkanes, preferably $C_3$-$C_6$ alkanes, to the corresponding methyl ketones, and converting secondary alcohols, preferably $C_3$-$C_7$ secondary alcohols, such as isopropanol, 2-butanol, 2-pentanol, 2-hexanol and 2-heptanol to the corresponding methyl ketones. The preferred alkenes herein are ethylene, propylene and 1-butene, most preferred being propylene, the preferred alcohols are isopropanol and 2-butanol, and the preferred alkanes are n-propane and n-butane.

The term "alicyclic and aromatic hydrocarbons" refers to saturated cycloaliphatic compounds such as cycloalkanes, benzene and alkaryl compounds combining aromatic rings with saturated alkyl groups such as, e.g., toluene, xylene and ethylbenzene. The preferred aromatic hydrocarbons are benzene and toluene.

As one embodiment of the present invention, there are provided compositions of matter comprising isolated and biologically pure microbial cultures of microorganisms utilizing $C_2$-$C_{10}$ alkyl compounds, as well as genetically engineered derivatives and natural mutants thereof, said cultures having the identifying

characteristics indicated hereinbelow and being capable of producing oxidative (monooxygenase and/or dehydrogenase) enzyme activity in isolatable amounts when cultured under aerobic conditions in a liquid growth medium comprising assimilable sources of nitrogen and essential mineral salts in the presence of a $C_2$-$C_{10}$ alkyl compound as the major carbon and energy source.

As another embodiment of the invention there is provided a process for producing the microbial cells which comprises culturing the microorganism strains under aerobic conditions in the liquid growth medium as described above in the presence of a $C_2$-$C_{10}$ alkyl compound.

As still another embodiment of the invention there is provided a process for advancing an oxidizable organic substrate containing at least two carbon atoms to a state of oxidation greater than its original state, which comprises contacting the substrate, in a reaction medium under aerobic conditions, with cells of a microorganism, a genetically engineered derivative or natural mutant thereof, or an enzyme preparation prepared from the cells or derivative, until at least a portion of the corresponding oxidation product is produced in isolatable amounts, wherein the microorganisms have been aerobically cultivated in a nutrient medium containing a $C_2$-$C_{10}$ alkyl compound and are selected from the newly discovered and isolated strains described below.

A preferred embodiment of the invention includes a process for producing 1,2-epoxides, most preferably propylene oxide, from $C_2$-$C_7$ alkenes, dienes or vinyl aromatic compounds, most preferably propylene, by contacting the indicated substrate under aerobic conditions with microbial cells or enzyme preparations thereof wherein the microbial cells are derived from the

newly discovered and isolated strains of the present invention as described below and have been previously grown under aerobic conditions in the presence of a $C_2$-$C_{10}$ alkane.

Another preferred embodiment of the invention includes a process for oxidizing $C_2$-$C_6$ alkanes, alicyclic hydrocarbons and aromatic hydrocarbons to the corresponding alcohols and/or ketones by contacting the indicated substrate under aerobic conditions with microbial cells or enzyme preparations thereof wherein the microbial cells are derived from the newly discovered and isolated strains herein previously grown under aerobic conditions in the presence of a $C_2$-$C_{10}$ alkane.

A third preferred embodiment herein includes a process for converting $C_3$-$C_7$ secondary alcohols, most preferably 2-butanol, to the corresponding methyl ketones by contacting the alcohol under aerobic conditions with microbial cells or enzyme preparations thereof wherein the microbial cells are derived from the newly discovered and isolated strains of the present invention as described below which have been previously grown under aerobic conditions in the presence of a $C_2$-$C_{10}$ alkane or a $C_2$-$C_{10}$ alkyl radical donating compound such as ethanol, propanol, butanol, propylamine, propyl formate, butyl formate, ethyl carbonate, propyl carbonate, etc.

The present invention includes the following features:

The new microorganism strains of this invention do not grow on methane and are morphologically and biochemically different from methane-utilizing bacteria.

Resting cell suspensions of the bacterial strains herein oxidize $C_2$-$C_5$ linear and branched alkenes, butadiene and isoprene to their corresponding 1,2-epoxides.

The product 1,2-epoxides are not further metabolized and accumulate extracellularly.

Among the substrate gaseous alkenes, propylene is oxidized at the highest rate.

Propane inhibits the epoxidation of propylene.

Both the hydroxylation and the epoxidation activities are located in the cell-free (enzyme extract) particulate fractions precipitated or sedimented between 10,000 x g and 40,000 x g centrifugation for one hour, when the cells were grown in shake flasks.

Cell-free particulate fractions from the microorganisms catalyze the epoxidation of $C_2$-$C_5$ n-alkenes and dienes (e.g., ethylene, propylene, 1-butene, 1-pentene, and butadiene) in the presence of oxygen and reduced nicotinamide adenine dinucleotide (NADH).

The cell-free soluble fraction (alkane monooxygenase) from a particular bacterial strain herein oxidizes $C_2$-$C_7$ linear and branched alkenes, butadiene and styrene to their corresponding 1,2-epoxides in the presence of oxygen and reduced nicotinamide adenine dinucleotide (NADH).

The epoxidation activities of the strains herein are strongly inhibited by various metal-binding agents.

Resting cell suspensions of the new microbes

herein oxidize (dehydrogenate) secondary alcohols to their corresponding methyl ketones. The product methyl ketones accumulate extracellularly. Among the secondary alcohols, isopropanol and 2-butanol are oxidized at the highest rate.

The rate of acetone and 2-butanone production from 2-propanol and 2-butanol, respectively, is linear for 120 minutes of incubation for the culture tested.

Cell suspensions of the strains grown on $C_2$-$C_4$ alkanes or $C_2$-$C_4$ alkyl radical donating compounds (e.g., propanol, butanol, propylamine, etc.) catalyze the conversion of secondary alcohols to the corresponding methyl ketones.

The cell-free particulate P(40) fractions derived from a particular strain of the invention convert secondary alcohols to the corresponding methyl ketones in the presence of oxygen and reduced nicotinamide adenine dinucleotide (NADH) as electron donor. This conversion is inhibited by various metal-binding agents, suggesting the involvement of metal(s) in the oxidation.

The cell-free soluble extracts derived from various strains of the invention catalyze an $NAD^+$ -dependent oxidation of secondary alcohols to the corresponding methyl ketones. This oxidation is inhibited by metal-binding agents and sulfhydryl inhibitors.

Alkanes, preferably $C_2$-$C_6$ alkanes, are converted to their corresponding alcohols and/or methyl ketones by cell suspensions of the strains of this invention. Of these alkanes, propane and n-butane are oxidized at the highest rate.

The oxidation products from branched alkanes and cyclopropane accumulate extracellularly.

Metal-chelating agents inhibit the production of methyl ketones from n-alkanes.

The cell-free particulate P(40) fractions derived from a particular strain of this invention catalyze oxygen- and reduced nicotinamide adenine dinucleotide ($NADH_2$) - dependent hydroxylation of n-alkanes.

The cell-free soluble fractions (alkane monooxygenase) from a particular strain herein oxidizes $C_2$-$C_6$ linear and branched alkanes, cyclohexane and toluene to their corresponding alcohols in the presence of oxygen and reduced nicotinamide adenine dinucleotide (NADH).

The newly discovered and isolated microorganism strains of the present invention were identified according to the classification system described in <u>Bergey's Manual of Determinative Bacteriology</u>, Robert S. Breed et al., eds., 8th ed. (Baltimore: Williams and Wilkins Co., 1974) and have the following designations:

<u>TABLE I</u>

| | Microorganism Strain Name | ER&E Designation | U.S.D.A. Agriculture Research Center Designation |
|---|---|---|---|
| 1. | *Acinetobacter* sp. | (CRL 67 P11) | NRRL B-11,313 |
| 2. | *Actinomyces* sp. | (CRL 66 P7) | NRRL 11,314 |
| 3. | *Arthrobacter* sp. | (CRL 60 P1) | NRRL B-11,315 |
| 4. | *Arthrobacter* sp. | (CRL 68 E1) | NRRL B-11,316 |
| 5. | *Arthrobacter* sp. | (CRL 70 B1) | NRRL B-11,317 |
| 6. | *Brevibacterium* sp. | (CRL 52 P3P) | NRRL B-11,318 |

| | | | |
|---|---|---|---|
| 7. | *Brevibacterium* sp. | (CRL 56 P6Y) | NRRL B-11,319 |
| 8. | *Brevibacterium* sp. | (CRL 61 P2) | NRRL B-11,320 |
| 9. | *Corynebacterium* sp. | (CRL 63 P4) | NRRL B-11,321 |
| 10. | *Mycobacterium* sp. | (CRL 51 P2Y) | NRRL B-11,322 |
| 11. | *Mycobacterium* sp. | (CRL 62 P3) | NRRL B-11,323 |
| 12. | *Mycobacterium* sp. | (CRL 69 E2) | NRRL B-11,324 |
| 13. | *Nocardia* sp. | (CRL 55 P5Y) | NRRL 11,325 |
| 14. | *Nocardia* sp. | (CRL 57 P7Y) | NRRL 11,326 |
| 15. | *Nocardia* sp. | (CRL 64 P5) | NRRL 11,327 |
| 16. | *Pseudomonas* sp. | (CRL 53 P3Y) | NRRL B-11,329 |
| 17. | *Pseudomonas* sp. | (CRL 54 P4Y) | NRRL B-11,330 |
| 18. | *Pseudomonas* sp. | (CRL 58 P9Y) | NRRL B-11,331 |
| 19. | *Pseudomonas* sp. | (CRL 65 P6) | NRRL B-11,332 |
| 20. | *Pseudomonas* sp. | (CRL 71 B2) | NRRL B-11,333 |

An important characteristic of the above-designated strains of the present invention is their capability to produce microbial cells when cultured under aerobic conditions in a liquid growth medium comprising assimilable sources of nitrogen and essential mineral salts containing a $C_2$-$C_{10}$ alkyl compound as their major carbon and energy source.

Each of the above-designated strains have been deposited at the United States Department of Agriculture, Agriculture Research Service, Northern Regional Research Laboratory (NRRL), Peoria, Illinois 61604 on the 26th June 1978 and have received from NRRL the individual NRRL designations as indicated above pursuant to a contract between NRRL and the assignee of this patent application, Exxon Research and Engineering Company (ER&E). The contract with NRRL provides for permanent availability of the progeny of these strains to the public upon the issuance of the U.S. patent describing and identifying the deposits or the publication or laying open to the public of any U.S. or foreign patent application or patent corresponding to this application, whichever occurs first. These strains

have however now been made available to the public from the 13th June 1983.

The assignee of the present application has agreed that, if any of these strains on deposit should die, or be lost or destroyed when cultivated under suitable conditions, it will be promptly replaced on notification with a viable culture of the same strain. It should be understood, however, that the availability of a deposit does not constitute a license to practise the subject invention in derogation of patent rights granted by governmental action.

The taxonomical and morphological characteristics of these newly isolated strains are shown below:

MORPHOLOGICAL AND TAXONOMICAL
CHARACTERISTICS OF MICROORGANISM STRAINS

1. *Acinetobacter* sp. CRL 67 strain P11 (NRRL B-11, 313) Cells are very short and plump in logarithmic growth phase, approaching coccus shape in stationary phase. Cells are predominantly in pairs and short chains. Non spore-forming, non-motile cells. Grow aerobically on $C_2$ to $C_{10}$ alkanes, $C_2$ to $C_{10}$ primary alcohols, propylamine, and nutrient agar. Do not grow on methane.

2. *Actinomyces* sp. CRL 66 strain P7 (NRRL 11,314) Organisms are gram-positive, irregular staining cells, non acid-fast, non spore-forming and non-motile. Many filamentous cells with

branching. Produce rough colonies on plate and pink pigmentation. Grow aerobically on $C_2$ to $C_{10}$ alkanes, $C_2$ to $C_{10}$ primary alcohols, propylamine and nutrient agar. Do not grow on methane.

3. Arthrobacter sp. CRL 60 strain P1 (NRRL B-11,315) Culture is composed of coccoid cells. In some old cultures, cells are spherical to ovoid or slightly elongated. Organisms are gram-positive, non acid-fast, strictly aerobic. Hydrolyze gelatin and reduce nitrate. Grow aerobically at the expense of $C_2$ to $C_{10}$ alkanes, $C_2$ to $C_{10}$ primary alcohols, propylamine, succinate and nutrient agar. Do not grow on methane.

4. Arthrobacter sp. CRL 68 strain E1 (NRRL B-11,316) Culture is composed of coccoid cells. In old cultures cells are spherical to ovoid or slightly elongated. Organisms are gram-positive, non acid-fast, strictly aerobic. Hydrolyze gelatin and reduce nitrate. Grow aerobically on $C_2$ to $C_{10}$ alkanes, $C_2$ to $C_{10}$ primary alcohols, ethylamine, propylamine, and nutrient agar. Do not grow on methane.

5. Arthrobacter sp. CRL 70 strain B1 (NRRL B-11,317) Culture is composed of coccoid cells. In some old cultures cells are spherical to ovoid or slightly elongated. Organisms are gram-positive, non-acid-fast, strictly aerobic. Hydrolyze gelatin and reduce nitrate. Grow aerobically on $C_2$ to $C_{10}$ alkanes, $C_2$ to $C_{10}$ primary alcohols, butylamine, and nutrient agar. Do not grow on methane.

6. Brevibacterium sp. CRL 52 P3P (NRRL B-11,318) Produce yellow, shiny raised colonies on mineral salt agar plates in the presence of $C_2$ to $C_{10}$ alkanes and primary alcohols. Also grow on nutrient media. Aerobic, rod-shaped organisms.

7. Brevibacterium sp. CRL 56 P6Y (NRRL B-11,319) Produce shiny, raised, yellow colonies on mineral salt agar plates in the presence of $C_2$ to $C_{10}$ alkanes and primary alcohols. Also grow on nutrient media. Aerobic, rod- to pear-shaped organisms.

8. Brevibacterium sp. CRL 61 strain P2 (NRRL B-11,320) Cells have coryneform morphology. They have "snapping" mode of cell division. Organisms are aerobic, forming yellowish to orange pigment on plates. Grow aerobically on $C_2$ to $C_{10}$ alkanes, $C_2$ to $C_{10}$ primary alcohols, propylamine, succinate and nutrient agar. Do not grow on methane.

9. Corynebacterium sp. CRL 63 strain P4 (NRRL B-11,321) Cells are straight or curved rods, frequently swollen at one or both ends. Gram-positive, usually stain unevenly and often contain metachromatic granules which stain bluish purple with methylene blue. Organisms liquify gelatin. Grow aerobically on $C_2$ to $C_{10}$ alkanes, $C_2$ to $C_{10}$ primary alcohols, propylamine, succinate and nutrient agar. Do not grow on methane.

10. Mycobacterium sp. CRL 51 P2Y (NRRL B-11,322) Produce small white colonies on mineral salt agar in the presence of $C_2$ to $C_{10}$ alkanes

and primary alcohols. Also grow on nutrient medium. The organisms are non-motile, gram-negative, aerobic rods.

11. *Mycobacterium* sp. CRL 62 strain P3 (NRRL B-11,323) Cells are short, plump rods, slightly curved with rounded or occasionally thickened ends. Acid-fast organism. Produce smooth, moist, shiny colonies with deep yellow pigmentation. Grow aerobically on $C_2$ to $C_{10}$ alkanes, $C_2$ to $C_{10}$ primary alcohols, propylamine, and nutrient agar. Do not grow on methane. Reduce nitrate and catalase test positive.

12. *Mycobacterium* sp. CRL 69 Strain E2 (NRRL B-11,324) Cells are short, plump rods, slightly curved with rounded or occasionally thickened rods. Acid-fast organism. Produce smooth, moist, shiny colonies with deep yellow pigmentation. Grow aerobically on $C_2$ to $C_{10}$ alkanes, $C_2$ to $C_{10}$ primary alcohols, ethylamine, propylamine and nutrient agar. Do not grow on methane.

13. *Nocardia* sp. CRL 55 P5Y (NRRL 11,325) Produce slow growing shiny, yellow colonies on mineral salt agar plates in the presence of $C_2$ to $C_{10}$ alkanes and primary alcohols. Also grown on nutrient media. Gram-positive, rod-shaped, aerobic organisms.

14. *Nocardia* sp. CRL 57 P7Y (NRRL 11,326) Produce shiny, raised cream- to yellow-colored colonies on mineral salt agar plates in the presence of $C_2$ to $C_{10}$ alkanes and primary alcohols. Also grow on nutrient media. Gram-positive, rod-shaped organisms.

15. Nocardia sp. CRL 64 strain P5 (NRRL 11,327) Colonies are dry and flaky. Cells produce branched mycelial which fragment into irregular bacillary and coccoid cells. Produce yellowish colonies on plates. Grow aerobically on $C_2$ to $C_{10}$ alkanes, $C_2$ to $C_{10}$ primary alcohols, propylamine, succinate and nutrient agar. Do not grow on methane.

16. Pseudomonas sp. CRL 53 P3Y (NRRL B-11,329) Produce small yellow colonies on mineral salt agar plates in the presence of $C_2$ to $C_{10}$ alkanes and primary alcohols. Also grow on nutrient media. Gram-negative, aerobic, motile, rod-shaped organisms.

17. Pseudomonas sp. CRL 54 P4Y (NRRL B-11,330) Produce shiny yellow colonies on mineral salt agar plates in the presence of $C_2$ to $C_{10}$ alkanes and primary alcohols. Also grow on nutrient media. Gram-negative, motile, aerobic rods.

18. Pseudomonas sp. CRL 58 P9Y (NRRL B-11,331) Produce yellow colonies on mineral salt agar plates in the presence of $C_2$ to $C_{10}$ alkanes and primary alcohols. Also grow on nutrient media. Gram-negative, motile, aerobic rods.

19. Pseudomonas sp. CRL 65 strain P6 (NRRL B-11,332) Organisms are gram-negative, aerobic rods. Motile by polar, monotrichous flagella. Do not produce flourescent pigment. Nitrate is denitrified by these organisms. Grow aerobically on $C_2$ to $C_{10}$ alkanes, $C_2$ to $C_{10}$ primary alcohols, propylamine and nutrient agar. Do not grow on methane.

20. Pseudomonas sp. CRL 71 strain B2 (NRRL B-11,333) Organisms are gram-negative, aerobic rods. Motile by polar monotrichous flagella. Do not produce flourescent pigment. Nitrate is denitrified by organisms. Grow aerobically on $C_2$ to $C_{10}$ alkanes, $C_2$ to $C_{10}$ primary alcohols, butylamine and nutrient agar. Do not grow on methane.

The newly dicovered and isolated strains of the present invention were obtained from soil samples from the Bayway Refinery in Linden, New Jersey, and from lake water samples from Warinanco Park, Linden, New Jersey. The samples were screened for the microorganisms by growth under oxygen and propane. The microorganisms were then isolated, purified and maintained by the procedure described below.

The maintenance of the cultures of these newly discovered and isolated strains should be carefully controlled. The preferred means for isolation and maintenance of the cultures is as follows. One gram of the soil or lake water samples is suspended in 10 ml of mineral salt medium as described below in Table II and allowed to settle at room temperature for one hour. The supernatant solution is inoculated into 300 ml flasks containing 50 ml of mineral salt medium.. The enrichment flasks are incubated at 30°C on a shaker under an atmosphere of gaseous ethane, propane or butane and air (1:1, vol./vol.). Within 96 hours the cultures become turbid. Serial dilutions of the enrichment cultures are prepared and spread onto mineral salt agar plates. These plates should be incubated in glass dessicators which have lids with an airtight seal and external sleeves with a tooled hose connection. Dessicators are to be evacuated and filled with a gas mixture of ethane, propane or butane and air (1:1 v/v).

Incubation should be at 30°C. Cultures will survive in these dessicators for three months at 4°C. However, frequent transfer of cultures is preferred.

## TABLE II

## MAINTENANCE OF CULTURES

The organisms are preferably subcultured every two weeks on mineral salts agar plates which contain medium having the following composition:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| KCl | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.14 g |
| $ZnSO_4$ | 0.02 mg |
| $MoO_3$ | 0.02 mg |
| Agar | 15 g |
| Water | 1 liter |

In commercial processes for the propagation of microorganisms, it is generally necessary to proceed by stages. These stages may be few or many, depending on the nature of the process and the characteristics of the microorganisms. Ordinarily, propagation is started by inoculating cells from a slant of a culture into a presterilized nutrient medium usually contained in a flask. In the flask, growth of the microorganisms is encouraged by various means, e.g., shaking for thorough aeration, and maintenance of suitable temperature. This step or stage is repeated one or more times in flasks

or vessels containing the same or larger volumes of nutrient medium. These stages may be conveniently referred to as culture development stages. The microorganisms with or without accompanying culture medium, from the last development stage, are introduced or inoculated into a large scale fermentor to produce commercial quantities of the microorganisms or enzymes therefrom.

Reasons for growing the microorganisms in stages are manifold, but are primarily dependent upon the conditions necessary for the growth of the microorganisms and/or the production of enzymes therefrom. These include stability of the microorganisms, proper nutrients, pH, osmotic relationships, degree of aeration, temperature and the maintenance of pure culture conditions during fermentation. For instance, to obtain maximum yields of the microbial cells, the conditions of fermentation in the final stage may have to be changed somewhat from those practised to obtain growth of the microorganisms in the culture development stages. Maintaining the purity of the medium, also, is an extremely important consideration, especially where the fermentation is performed under aerobic conditions as in the case of the microorganisms herein. If the fermentation is initially started in a large fermentor, a relatively long period of time will be needed to achieve an appreciable yield of microorganisms and/or oxidative and dehydrogenase enzymes therefrom. This, of course, enhances the possibility of contamination of the medium and mutation of the microorganisms.

The culture media used for growing the microorganisms and inducing the oxidative enzyme system will be comprised of inorganic salts of phosphate, sulfates, and nitrates as well as oxygen and a source of $C_2$-$C_{10}$ alkyl compounds. The fermentation will generally be

conducted at temperatures ranging from 5° to about 50°C, preferably at temperatures ranging from about 25° to about 45°C. The pH of the culture medium should be controlled at a pH ranging from about 4 to 9, and preferably from about 5.5 to 8.5, and more preferably from 6.0 to 7.5. The fermentation may be conducted at atmospheric pressures, although higher pressures up to about 5 atmospheres and higher may be employed.

Typically, to grow the microorganisms and to induce the oxygenase and dehydrogenase enzymes, the microorganisms are inoculated into the medium which is contacted with a gas mixture containing the $C_2$-$C_{10}$ alkyl compound and oxygen. For continuous flow culture the microorganisms may be grown in any suitably adapted fermentation vessel, for example, a stirred baffled fermentor or sparged tower fermentor, which is provided either with internal cooling or an external recycle cooling loop. Fresh medium may be continuously pumped into the culture at rates equivalent to 0.02 to 1 culture volume per hour and the culture may be removed at a rate such that the volume of culture remains constant. A gas mixture containing the $C_2$-$C_{10}$ alkyl compound and oxygen and possibly carbon dioxide or other gases is contacted with the medium preferably by bubbling continuously through a sparger at the base of the vessel. The source of oxygen for the culture may be air, oxygen or oxygen-enriched air. Spent gas may be removed from the head of the vessel. The spent gas may be recycled either through an external loop or internally by means of a gas inducer impeller. The gas flow rate and recycling should be arranged to give maximum growth of microorganism and maximum utilization of the $C_2$-$C_{10}$ alkyl compound.

The oxygenase enzyme system may be obtained as a crude extract, or as a cell-free particulate or

soluble fraction. When it is desired to obtain the secondary alcohol dehydrogenase (SADH) enzyme fraction one first breaks the cells, e.g., sonication, etc., and then removes the cellular debris, e.g., centrifuges at 30,000 x g. for about 30 minutes. The microbial cells may be harvested from the growth medium by any of the standard techniques commonly used, for example, flocculation, sedimentation, and/or precipitation, followed by centrifugation and/or filtration. The biomass may be dried, e.g, by freeze or spray drying and may be used in this form for further use in the oxidation reactions.

To put the invention to practice, the microorganism cells or an oxygenase or dehydrogenase enzyme preparation prepared from the cells is employed which will convert oxidizable organic substrates to their oxidized products under aerobic conditions. One obtains such a preparation from any of the microorganism strains (or natural and/or artificial mutant thereof) described above and preferably grows the microorganism in a nutrient medium containing a $C_2$-$C_{10}$ alkyl compound and oxygen as described above. The nutrient medium is preferably the culture medium described by Foster and Davis, J. Bacteriol., 91, 1924 (1966).

The enzyme preparation is then brought into contact with the desired oxidizable substrate, e.g., a $C_2$-$C_7$ alkene or diene, such as ethylene, propylene, 1-butene, cis-but-2-ene, trans-but-2-ene, isobutene, 1-pentene, 1-hexene, 1-heptene, butadiene, isoprene or mixtures thereof, a vinyl aromatic compound such as styrene, an alicyclic compound such as cyclopropane or cyclohexane, an alkane such as ethane, propane, butane, isobutane, etc., an aromatic compound such as benzene or toluene, or a linear secondary alcohol, e.g., 2-propanol or 2-butanol in the presence of oxygen and a buffer solution, and the mixture is incubated until the desired

degree of conversion has been obtained. Thereafter, the oxidized product is recovered by conventional means, e.g., distillation, etc. Preferably, the oxidation is carried out at a temperature in the range from about 5 to about 55°C, more preferably about 25 to about 50°C, and at a pH in the range from about 4 to about 9, more preferably 5.5 to 8.5.

To facilitate the necessary effective contact of oxygen and the enzyme (whether it be an enzyme preparation or cells of a microorganism), it is preferred, for best results, to employ a strong, finely divided air stream into a vigorously stirred dispersion of substrate in the oxidation medium which generally contains water and a buffer and in which the enzyme preparation or microorganism culture is suspended. The enzyme preparation may then be separated from the liquid medium, preferably by filtration or centrifugation. The resulting oxidized product may then generally be obtained.

The process of the invention may be carried out batchwise, semi-continuously, continuously, concurrently or countercurrently. Optionally, the suspension containing the enzyme preparation or microorganism cells and buffer solution is passed downwardly with vigorous stirring countercurrently to an air stream rising in a tube reactor. The top layer is removed from the downflowing suspension, while culture and remaining buffer solution constituents are recycled, at least partly, with more oxidative substrate and addition of fresh enzyme preparation or microorganism cells as required.

The growth of the microorganisms and the oxidation process may be conveniently coupled by conducting them simultaneously, but separately and using much higher aeration in the oxidation process (e.g., an

air excess of at least twice that required for growth, preferably at least five times as much aeration). Both the growth process and the oxidation processes may be conducted in the same reactor in sequential or simultaneous operations by alternate use of normal and strong aeration.

As described above, the newly discovered and isolated microorganisms of the present invention grow well under aerobic conditions in a nutrient medium containing $C_2$-$C_{10}$ carbon-containing compounds. When these carboncontaining compounds are oxygenase and/or dehydrogenase enzyme inducers, the resulting resting microbial cells and/or their enzyme preparations are capable of increasing the oxidative state of a plurality of oxidizable substrates.

As will be shown by the examples that follow, the $C_2$-$C_{10}$ alkyl compound-grown microbial cells and their enzyme preparations (including cell-free extracts) possess oxygenase and/or dehydrogenase enzyme activity so as to oxidize saturated hydrocarbons, olefins, and secondary alcohols to their respective corresponding oxidized product.

The invention is illustrated further by the following examples which, however, are not to be taken as limiting in any respect. All parts and percentages, unless expressly stated otherwise, are by weight.

## EXAMPLE 1

### Cell Suspensions:

Growth, Preparation and Oxidation Procedures

A nutrient medium as described by Foster and

Davis, J. Bacteriol., 91, 1924 (1966), supra, having the following composition per liter of water was prepared:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| KCl | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.02 mg |
| $ZnSO_4$ | 0.14 mg |
| $MoO_3$ | 0.02 mg |

The pH of the nutrient medium was adjusted to 7.0 by the addition of acid or base and 50 ml samples of the nutrient medium were charged into a plurality of 300 ml shake flasks. The shake flasks were inoculated with an innoculating loop of cells from an agar plate containing homogeneous colonies of the microorganisms on the plate (the purity of the isolates was confirmed by microscopic examination). The isolates had been maintained on agar plates in a desiccator jar under an atmosphere of $C_2$-$C_4$ alkane (i.e. ethane, propane or butane) and air having a 1:1 v/v gas ratio which had been transferred every two weeks. The gaseous phase of the inoculated flasks was then replaced with a gas mixture comprised of a $C_2$-$C_4$ alkane and air having a ratio of 1:1 on a v/v basis. In the case of growth on liquid substrates (at 0.3%, v/v), the gaseous phase of the flasks was air. The inoculated flasks were sealed air tight and were incubated on a rotary shaker at 250 rpm and at 30°C for two days until turbidity in the medium had developed.

The cells were harvested by centrifugation at 12,000 x g. at 4°C for 15 minutes. The cell pellet was washed twice with a 50 mM phosphate buffer at a pH of 7.0. The washed cells were then suspended in a 50mM phosphate buffer at pH 7.0.

A 0.5 ml sample of each washed cell suspension (2 mg cells) was put into separate 10 ml vials at 4°C which were sealed with a rubber cap. The gaseous phase of the vials was removed with vacuum and then was replaced with a gas mixture of the oxidative substrate (e.g. ethane) and oxygen at a 1:1 v/v ratio. If a liquid substrate was employed, 5-10 μl of substrate was placed in the vials. The vials were then incubated at 30°C on a rotary shaker at 200 rpm. Samples (3 μl) were withdrawn periodically with a microsyringe and the products were analyzed by gas chromatography (ionization flame detector column) at a column temperature of 100°C to 180°C and at a carrier gas flow rate of 30 or 35 ml/min of helium. The various products were identified by retention time comparisons and co-chromatography with authentic standards. The amount of product formed was determined from the peak area using a standard graph which had been constructed with an authentic standard. Protein in the cell suspensions was determined by the method described by O. H. Lowry et al., J. Biol. Chem., 193, 265 (1951).

EXAMPLE 2

Epoxidation of Alkenes to 1,2-Epoxides

The newly discovered and isolated microorganism strains of the present invention were each grown aerobically in the presence of the alkane growth substrate indicated in Table III in the manner described in Example 1. The microbial cells were then washed,

recovered and put to use in epoxidizing an alkene or diene substrate using the procedure described above, where in the case of a liquid substrate 10 $\mu l$ of the substrate was used. Table III shows the results of these experiments where the oxidative substrates were ethylene, propylene, 1-butene, butadiene, 1-pentene and 1-hexene.

EXAMPLE 3

Microbiological Conversion of Alkanes to Ketones

The newly discovered and isolated microorganism strains of the present invention were each grown aerobically in the presence of the alkane growth substrate indicated in Table IV in the manner described in Example 1. The microbial cells were then washed, recovered and contacted with an oxidative alkane substrate using the oxidation procedure of Example 1. Table IV shows the results of these experiments where the oxidative substrates were propane, butane, pentane, hexane and heptane, which were converted to their respective ketone products.

## TABLE III

Epoxidation of Alkenes by Microorganisms Grown on Gaseous Alkanes

| Microorganism Strain Identification[a] | Growth Substrate | Oxidative Conversion Rates ($\mu$mole/hr/mg protein)[b] | | | | | |
|---|---|---|---|---|---|---|---|
| | | Ethylene to Ethylene Oxide | Propylene to Propylene Oxide | 1-Butene to 1,2-Epoxy-Butane | 1,3-Butadiene to 1,2-Epoxy-Butane | 1-Pentene to 1,2-Epoxy-Pentane | 1-Hexene to 1,2-Epoxy Hexane |
| *Acinetobacter* sp. (CRL 67 P11) NRRL B-11,313 | propane | 0.20 | 1.2 | 1.20 | 0.02 | 0.020 | 0.002 |
| *Actinomyces* sp. (CRL 66 P7) NRRL 11,314 | propane | 0.90 | 1.0 | 0.97 | 0.018 | 0.011 | 0.007 |
| *Arthrobacter* sp. (CRL 60 P1) NRRL B-11,315 | propane | 1.9 | 2.5 | 0.10 | 0.025 | 0.01 | 0.01 |
| *Arthrobacter* sp. (CRL 68 E1) NRRL B-11,316 | ethane | 0.49 | 1.7 | 0.12 | 0.06 | 0.06 | - |
| *Arthrobacter* sp. (CRL 70 B1) NRRL B-11,317 | butane | 1.2 | 0.59 | 0.18 | 0.03 | 0.02 | - |
| *Brevibacterium* sp. (CRL 52 P3P) NRRL B-11,318 | propane | 1.7 | 2.1 | 0.48 | 0.04 | 0.03 | - |
| *Brevibacterium* sp. (CRL 56 P6Y) NRRL B-11,319 | propane | 0.98 | 2.4 | 0.28 | 0.03 | 0.02 | - |

TABLE III (Cont'd)

| Microorganism Strain Identification(a) | Growth Substrate | Oxidative Conversion Rates (μ mole/hr/mg protein)(b) Ethylene to Ethylene Oxide | Propylene to Propylene Oxide | 1-Butene to 1,2-Epoxy-Butane | 1,3-Butadiene to 1,2-Epoxy-Butane | 1-Pentene to 1,2-Epoxy-Pentane | 1-Hexene to 1,2-Epoxy Hexane |
|---|---|---|---|---|---|---|---|
| Brevibacterium sp. | | | | | | | |
| (CRL 61 P2) NRRL B-11,320 | ethane | 1.7 | 0.21 | 0.20 | 0.062 | 0.01 | 0.005 |
| (CRL 61 P2) NRRL B-11,320 | propane | 2.1 | 2.8 | 0.15 | 0.032 | 0.09 | 0.009 |
| (CRL 61 P2) NRRL B-11,320 | butane | 1.0 | 0.50 | 1.9 | 0.028 | 0.016 | 0.010 |
| Corynebacterium sp. (CRL 63 P4) NRRL B-11,321 | propane | 0.25 | 2.4 | 1.7 | 0.98 | 0.025 | 0.01 |
| Mycobacterium sp. (CRL 51 P2Y) NRRL B-11,322 | propane | 0.52 | 0.62 | 0.25 | 0.07 | 0.03 | 0 |
| Mycobacterium sp. (CRL 62 P3) NRRL B-11,323 | propane | 0.30 | 0.50 | 0.18 | 0.075 | 0.02 | 0.012 |
| Mycobacterium sp. (CRL 69 E2) NRRL B-11,324 | ethane | 0.82 | 0.90 | 0.24 | 0.052 | 0.05 | 0.002 |
| Nocardia sp. (CRL 55 P5Y) NRRL 11,325 | propane | 0.20 | 0.92 | 0.68 | 0.42 | 0.02 | 0 |
| Nocardia sp. (CRL 57 P7Y) NRRL 11,326 | propane | 0.26 | 2.0 | 1.2 | 0.98 | 0.08 | 0.01 |
| Nocardia sp. (CRL 64 P5) NRRL 11,327 | propane | 0.28 | 2.0 | 1.4 | 0.92 | 0.081 | 0.008 |

TABLE III (Cont'd)

| Microorganism Strain Identification(a) | Growth Substrate | Oxidative Conversion Rates ($\mu$ mole/hr/mg protein)(b) Ethylene to Ethylene Oxide | Propylene to Propylene Oxide | 1-Butene to 1,2-Epoxy-Butane | 1,3-Butadiene to 1,2-Epoxy-Butane | 1-Pentene to 1,2-Epoxy-Pentane | 1-Hexene to 1,2-Epoxy Hexane |
|---|---|---|---|---|---|---|---|
| Pseudomonas sp. | | | | | | | |
| (CRL 53 P3Y) NRRL B-11,329 | ethane | 0.50 | 1.20 | 0.10 | 0.05 | 0.02 | 0 |
| (CRL 53 P3Y) NRRL B-11,329 | propane | 1.00 | 1.20 | 0.92 | 0.03 | 0.04 | - |
| (CRL 53 P3Y) NRRL B-11,329 | butane | 0.30 | 0.80 | 1.00 | 1.40 | 0.10 | 0.04 |
| Pseudomonas sp. (CRL 54 P4Y) NRRL B-11,330 | propane | 0.90 | 1.10 | 0.90 | 0.02 | 0.02 | 0 |
| Pseudomonas sp. (CRL 58 P9Y) NRRL B-11,331 | propane | 0.80 | 1.00 | 0.80 | 0.02 | 0.02 | 0 |
| Pseudomonas sp. (CRL 65 P6) NRRL B-11,332 | propane | 1.1 | 0.41 | 0.90 | 0.012 | 0.009 | 0.005 |
| Pseudomonas sp. (CRL 71 B2) NRRL B-11,333 | butane | 2.3 | 3.5 | 0.18 | 0.03 | 0.020 | - |

(a) The dry weight of the cells was about 0.2g/100 ml culture broth.

(b) The products of microbial oxidation were identified by gas chromatographic retention time with authentic standards. The identification of the products was supplemented by establishing the presence or absence of product peaks before and after bromination or acid hydrolysis. Analysis also revealed that no further oxidation of epoxide product occurred.

**TABLE IV**

**Microbiological Conversion of Alkanes to Ketones**

| Microorganism Strain Identification | Growth Substrate | Oxidative Conversion Rates ($\mu$ mole/hr/mg protein): Propane to Acetone | Butane to 2-Butanone | Pentane to 2-Pentanone | Hexane to 2-Hexanone | Heptane to 2-Heptanone |
|---|---|---|---|---|---|---|
| *Acinetobacter* sp. (CRL 67 P11) NRRL B-11,313 | propane | 0.9 | 0.75 | 0.15 | 0.03 | 0.005 |
| *Actinomyces* sp. (CRL 66 P7) NRRL 11,314 | propane | 1.2 | 0.8 | 0.10 | 0.02 | 0.015 |
| *Arthrobacter* sp. (CRL 60 P1) NRRL B-11,315 | propane | 1.4 | 2.6 | 0.21 | 0.12 | 0.009 |
| *Arthrobacter* sp. (CRL 68 E1) NRRL B-11,316 | ethane | 1.8 | 2.1 | 0.39 | 0.11 | 0.017 |
| *Arthrobacter* sp. (CRL 70 B1) NRRL B-11,317 | ethane<br>butane | 1.8<br>2.9 | 2.1<br>2.0 | 0.39<br>0.28 | 0.11<br>0.08 | 0.015<br>0.021 |
| *Brevibacterium* sp. (CRL 52 P3P) NRRL B-11,318 | propane | 1.7 | 1.2 | 0.30 | 0.12 | 0.010 |
| *Brevibacterium* sp. (CRL 56 P6Y) NRRL B-11,319 | propane | 2.0 | 2.0 | 0.20 | 0.06 | ND |
| *Brevibacterium* sp. (CRL 61 P2) NRRL B-11,320 | propane | 2.5 | 2.0 | 0.12 | 0.09 | 0.015 |

TABLE IV (Cont'd)

| Microorganism Strain Identification | Growth Substrate | Oxidative Conversion Rates (μ mole/hr/mg protein) Propane to Acetone | Butane to 2-Butanone | Pentane to 2-Pentanone | Hexane to 2-Hexanone | Heptane to 2-Heptanone |
|---|---|---|---|---|---|---|
| *Corynebacterium* sp. (CRL 63 P4) NRRL B-11,321 | propane | 3.5 | 3.1 | 0.18 | 0.09 | 0.012 |
| *Mycobacterium* sp. (CRL 51 P2Y) NRRL B-11,322 | propane | 1.1 | 1.6 | 0.23 | 0.07 | 0.09 |
| *Mycobacterium* sp. (CRL 62 P3) NRRL B-11,323 | propane | 1.2 | 1.8 | 0.20 | 0.07 | 0.007 |
| *Mycobacterium* sp. (CRL 69 E2) NRRL B-11,324 | ethane | 1.4 | 0.9 | 0.48 | 0.09 | 0.019 |
| *Nocardia* sp. (CRL 55 P5Y) NRRL 11,325 | propane | 1.9 | 2.1 | 0.17 | 0.05 | 0.015 |
| *Nocardia* sp. (CRL 57 P7Y) NRRL 11,326 | propane | 1.2 | 1.8 | 0.10 | ND | ND |
| *Nocardia* sp. (CRL 64 P5) NRRL 11,327 | propane | 1.8 | 2.5 | 0.19 | 0.07 | 0.015 |
| *Pseudomonas* sp. (CRL 53 P3Y) NRRL B-11,329 | propane | 4.0 | 1.1 | 0.31 | 0.03 | 0.009 |

TABLE IV (Cont'd)

| Microorganism Strain Identification | Growth Substrate | Oxidative Conversion Rates (μmole/hr/mg protein): Propane to Acetone | Butane to 2-Butanone | Pentane to 2-Pentanone | Hexane to 2-Hexanone | Heptane to 2-Heptanone |
|---|---|---|---|---|---|---|
| *Pseudomonas* sp. (CRL 54 P4Y) NRRL B-11,330 | propane | 0.9 | 1.0 | 0.10 | ND | ND |
| *Pseudomonas* sp. (CRL 58 P9Y) NRRL B-11,331 | propane | 1.2 | 1.2 | 0.16 | ND | ND |
| *Pseudomonas* sp. (CRL 65 P6) NRRL B-11,332 | propane | 4.2 | 1.2 | 0.20 | 0.021 | 0.008 |
| *Pseudomonas* sp. (CRL 71 B2) NRRL B-11,333 | butane | 2.8 | 1.8 | 0.42 | 0.28 | 0.015 |

ND = not determined

One strain, Arthrobacter sp. (CRL 60 P1) NRRL B-11,315, grown on propane as described in Example 1, was used to convert n-propane, n-butane, n-pentane, and n-hexane to their respective 2-alcohols using the procedure described in Example 1. The conversion rates in $\mu$ moles/hr/5 mg protein are indicated below.

| n-Propane to 2-Propanol | n-Butane to 2-Butanol | n-Pentane to 2-Pentanol | n-Hexane to 2-Hexanol |
|---|---|---|---|
| 0.12 | 0.09 | 0.007 | 0.003 |

## EXAMPLE 4

### Microbiological Conversion of Cycloalkanes and Aromatic Compounds

The procedure of Example 1 was repeated wherein a plurality of the newly discovered and isolated strains were each grown aerobically in a nutrient medium containing the indicated growth substrate. The washed cells of the microorganisms were then contacted with cyclopropane (using one strain only), cyclohexane, benzene or toluene using the oxidation procedure of Example 1. The reaction products were analyzed and found to contain cyclohexanol, phenol or benzyl alcohol, respectively. The results of this series of experiments are shown in Table V.

TABLE V

Microbiological Oxidation of Cycloalkanes and Aromatic Compounds

| Microorganism Strain Identification | Growth Substrate | Oxidative Conversion Rates (µ mole/hr/mg protein) Cyclopropane to Cyclopropanol | Cyclohexane to Cyclohexanol | Benzene to Phenol | Toluene to Benzyl Alcohol |
|---|---|---|---|---|---|
| *Arthrobacter* sp. (CRL 60 P1) NRRL B-11,315 | propane<br>butane | ND<br>ND | 0<br>1.4 | 0.11<br>2.3 | 0<br>0.40 |
| *Brevibacterium* sp. (CRL 56 P6Y) NRRL B-11,319 | propane | 0.038 | 1.03 | 0.30 | 0 |
| *Brevibacterium* sp. (CRL 61 P2) NRRL B-11,320 | ethane | ND | 1.1 | 1.2 | 0.01 |
| *Corynebacterium* sp. (CRL 63 P4) NRRL B-11,321 | propane | ND | 0.1 | 0.23 | 0.05 |
| *Mycobacterium* sp. (CRL 51 P2Y) NRRL B-11,322 | propane | ND | 0 | 2.0 | 0 |
| *Pseudomonas* sp. (CRL 53 P3Y) NRRL B-11,329 | propane | ND | 4.7 | 0.75 | 0.06 |
| *Pseudomonas* sp. (CRL 58 P9Y) NRRL B-11,331 | propane | ND | 0.42 | 0.07 | 0.07 |

ND = not determined

(a) Only one strain was used for this conversion.

## EXAMPLE 5

### Microbiological Conversion of Branched Alkanes, Branched Alkenes and Branched Dienes

The procedure of Example 1 was repeated wherein *Brevibacterium* sp. (CRL 56 P6Y) NRRL B-11,319 was grown aerobically in a nutrient medium containing propane. The washed cells of the microorganisms were then contacted with isobutane, 2,2-dimethylpropane, isobutene, 2-methyl-1-butene and isoprene using the oxidation procedure of Example 1. The results of this series of experiments are shown in Table VI.

TABLE VI

Microbiological Oxidation of Branched Alkanes, Branched Alkenes and Branched Dienes

| Substrate | Product | Oxidative Conversion Rates ($\mu$moles/hr/mg protein) |
|---|---|---|
| Isobutane | tert-butanol | 0.102 |
| 2,2-Dimethylpropane | ND | 0.027 |
| Isobutene | 1,2-epoxyisobutene | 0.205 |
| 2-Methyl-1-butene | 2-methyl-1,2-epoxy-butane | 0.195 |
| Isoprene | 1,2-epoxyisoprene | 0.010 |

ND = not determined

The optimum pH and temperature for these oxidation reactions were found to be about 7.0 and 30°C, respectively.

The reactions proceeded linearly for up to two hours and the products accumulated extracellularly. Cyclopropane from Example 4 behaved in a similar manner.

## EXAMPLE 6

## Microbiological Conversion of Secondary Alcohols to Ketones

The procedure of Example 1 was repeated wherein the newly discovered and isolated microorganism strains of the present invention were each grown aerobically in the presence of the indicated gaseous alkane or $C_2$-$C_4$ alkyl radical donating compound. The washed cells of the microorganisms were then contacted with secondary alcohols, i.e., isopropanol, 2-butanol, 2-pentanol, 2-hexanol, or 2-heptanol using the oxidation procedure of Example 1, to produce the corresponding ketone products. The results of these experiments are shown in Table VII.

**TABLE VII**

**Microbiological Conversion of Sec. Alcohols to Ketones**

| Microorganism - Strain Identification | Growth Substrate | Oxidative Conversion Rates ( μ mole/hr/mg protein) | | | | |
|---|---|---|---|---|---|---|
| | | Isopropanol to Acetone | 2-Butanol to 2-Butanone | 2-Pentanol to 2-Pentanone | 2-Hexanol to 2-Hexanone | 2-Heptanol to 2-Heptanone |
| *Acinetobacter* sp. (CRL 67 P11) NRRL B-11,313 | propane | 2.4 | 3.5 | 0.32 | 0.18 | - |
| *Actinomyces* sp. (CRL 66 P7) NRRL 11,314 | propane | 2.1 | 3.5 | 0.25 | 0.12 | 0.027 |
| | propanol | 2.2 | 3.5 | 0.46 | 0.16 | 0.029 |
| *Arthrobacter* sp. (CRL 60 P1) NRRL B-11,315 | propane | 2.2 | 5.0 | 0.42 | 0.18 | 0.02 |
| | propanol | 2.4 | 5.5 | 0.52 | 0.19 | 0.042 |
| | propylamine | 1.95 | 5.1 | 0.48 | 0.27 | 0.025 |
| *Arthrobacter* sp. (CRL 68 E1) NRRL B-11,316 | ethane | 0.98 | 3.2 | 0.49 | 0.18 | 0.017 |
| | ethanol | 3.1 | 3.5 | 0.52 | 0.30 | 0.021 |
| | ethylamine | 2.7 | 2.9 | 0.29 | 0.31 | 0.012 |
| *Arthrobacter* sp. (CRL 70 B1) NRRL B-11,317 | butane | 2.8 | 4.7 | 0.48 | 0.27 | 0.021 |
| | butanol | 2.8 | 5.2 | 0.54 | 0.32 | 0.012 |
| | butylamine | 3.0 | 4.1 | 0.41 | 0.28 | 0.017 |
| *Brevibacterium* sp. (CRL 52 P3P) NRRL B-11,318 | propane | 3.1 | 5.7 | 0.57 | 0.31 | 0.032 |
| | propanol | 3.3 | 6.2 | 1.0 | 0.39 | 0.031 |

TABLE VII (Cont'd)

| Microorganism Strain Identification | Growth Substrate | Oxidative Conversion Rates (μ mole/hr/mg protein) Isopropanol to Acetone | 2-Butanol to 2-Butanone | 2-Pentanol to 2-Pentanone | 2-Hexanol to 2-Hexanone | 2-Heptanol to 2-Heptanone |
|---|---|---|---|---|---|---|
| *Brevibacterium sp.* (CRL 56 P6Y) NRRL B-11,319 | propane | 3.0 | 5.1 | 0.51 | 0.30 | 0.019 |
| *Brevibacterium sp.* (CRL 61 P2) NRRL B-11,320 | ethane | 2.1 | 2.5 | 0.52 | 0.31 | 0.031 |
| | propane | 2.1 | 5.2 | 0.48 | 0.25 | 0.015 |
| | butane | 3.0 | 4.1 | 0.30 | 0.21 | 0.048 |
| | ethanol | 1.2 | 4.2 | 0.31 | 0.25 | - |
| | propanol | 2.2 | 5.7 | 0.54 | 0.27 | 0.019 |
| | butanol | 2.5 | 4.0 | 0.47 | 0.25 | 0.051 |
| | propylamine | 2.1 | 3.2 | 0.32 | 0.21 | 0.031 |
| *Corynebacterium sp.* (CRL 63 P4) NRRL B-11,321 | propane | 8.5 | 7.5 | 0.32 | 0.25 | 0.032 |
| | propanol | 8.8 | 7.8 | 0.41 | 0.15 | 0.037 |
| *Mycobacterium sp.* (CRL 51 P2Y) NRRL B-11,322 | propane | 2.9 | 4.5 | 1.1 | 0.45 | 0.021 |
| | propanol | 3.1 | 4.8 | 0.35 | 0.12 | 0.021 |
| *Mycobacterium sp.* (CRL 62 P3) NRRL B-11,323 | propane | 2.8 | 4.0 | 0.98 | 0.31 | 0.018 |
| | propanol | 3.5 | 4.5 | 1.1 | 0.47 | 0.025 |
| *Mycobacterium sp.* (CRL 69 E2) NRRL B-11,324 | ethane | 1.6 | 3.0 | 0.38 | 0.09 | 0.029 |
| | ethanol | 2.5 | 3.1 | 0.41 | 0.12 | 0.030 |

TABLE VII (Cont'd)

| Microorganism Strain Identification | Growth Substrate | Oxidative Conversion Rates (μ mole/hr/mg protein) Isopropanol to Acetone | 2-Butanol to 2-Butanone | 2-Pentanol to 2-Pentanone | 2-Hexanol to 2-Hexanone | 2-Heptanol to 2-Heptanone |
|---|---|---|---|---|---|---|
| *Nocardia* sp. (CRL 55 P5Y) NRRL 11,325 | propane | 2.5 | 5.7 | 0.42 | 0.21 | 0.051 |
| | propanol | 2.1 | 5.1 | 0.43 | 0.22 | 0.053 |
| *Nocardia* sp. (CRL 57 P7Y) NRRL 11,326 | propane | 5.8 | 7.2 | ND | ND | ND |
| *Nocardia* sp. (CRL 64 P5) NRRL 11,327 | propane | 2.0 | 5.2 | 0.30 | 0.19 | 0.045 |
| | propanol | 2.5 | 5.9 | 0.45 | 0.35 | 0.059 |
| *Pseudomonas* sp. (CRL 53 P3Y) NRRL B-11,329 | propane | 6.1 | 4.8 | 0.35 | 0.18 | 0.045 |
| | propanol | 6.2 | 4.9 | 0.39 | 0.18 | 0.045 |
| *Pseudomonas* sp. (CRL 54 P4Y) NRRL B-11,330 | propane | 8.0 | 8.8 | 0.84 | ND | ND |
| *Pseudomonas* sp. (CRL 58 P9Y) NRRL B-11,331 | propane | 10.0 | 9.4 | 0.96 | ND | ND |
| *Pseudomonas* sp. (CRL 65 P6) NRRL B-11,332 | propane | 6.2 | 4.2 | 0.29 | 0.16 | 0.07 |
| | propanol | 6.5 | 4.5 | 0.35 | 0.18 | 0.081 |
| *Pseudomonas* sp. (CRL 71 B2) NRRL B-11,333 | butane | 2.1 | 2.8 | 0.42 | 0.25 | 0.050 |
| | butanol | 2.7 | 3.0 | 0.40 | 0.21 | - |

EXAMPLE 7

Effect of Inhibitors on Oxidation Activity

It has been reported that the oxidation of methane and propylene by cell suspensions and P(40) particulate fractions of methane-utilizing bacteria was inhibited by various metal-binding or metal chelating agents (see, e.g., U.S. Pat. No. 4,266,034). Hence, the effect of inhibitors on propylene- and propane-oxidizing activities by propane-grown cell suspensions of Arthrobacter sp. (CRL 60 P1) NRRL B-11,315 was examined. The production of acetone and propylene oxide was inhibited by various metal-binding compounds with different ligand combinations, i.e., nitrogen-nitrogen ( $\alpha,\alpha$ -bipyridyl), oxygen-nitrogen (8-hydroxyquinoline), and sulfur-nitrogen (thiourea, thiosemicarbazide) as shown in Table VIII. All inhibitors were added at 1mM concentration and the cell suspensions were incubated for 15 min. at 4°C.

TABLE VIII

Effect of Inhibitor on Propylene- and Propane-Oxidizing Activity of *Arthrobacter* sp. (CRL 60 Pl) NRRL B-11,315

| Inhibitor | Inhibition (%) Propylene-Oxidizing Activity[(a)] | Inhibition (%) Propane-Oxidizing Activity[(b)] |
|---|---|---|
| Thiourea | 12 | 10 |
| Thiosemicarbazide | 12 | 10 |
| 1,10-Phenanthroline | 90 | 80 |
| α,α-Bipyridyl | 32 | 30 |
| Potassium cyanide | 54 | 60 |
| Hydroxylamine | 83 | 70 |
| Isoniazide | 35 | 30 |
| p-Hydroxymercuribenzoate | 35 | 35 |
| N-Ethylmaleimide | 81 | 70 |
| Thioacetamide | 68 | 60 |
| Imidazole | - | 80 |

(a) The propylene-oxidizing activity was estimated by measuring propylene oxide formation by gas chromatography as described in Example 1. The uninhibited rate of propylene oxide production was 0.6 μmoles/15 min/mg of protein in cell suspensions.

(b) The propane-oxidizing activity was estimated by measuring the production of acetone by gas chromatography as described in Example 1. The uninhibited rate of acetone production was 1.5 μ moles/hr/mg of protein in cell suspensions.

EXAMPLE 8

Optimal Conditions for Epoxidation

The following summarizes tests conducted on the optimal conditions for the production of propylene oxide from propylene using cell suspensions of propane-grown *Arthrobacter* sp. (CRL 60 P1) NRRL B-11,315 as the representative microorganism strain. It will be understood that these are optimal conditions found for one particular strain and one particular substrate, and the invention is not meant to be bound by them. Conversions of propylene can still be obtained using *Arthrobacter* sp. (CRL 60 P1) NRRL B-11,315 by deviating from the optimum indicated below, but with lower yields and conversions.

Time Course

The production of propylene oxide from propylene reached a maximum after 2 to 3 hours of incubation in batch experiments. The rate of propylene oxide formation was linear for the first 40 minutes of incubation at $30^{\circ}C$. Therefore, the production of epoxide was measured within 30 minutes of incubation whenever the effect of a variable was tested.

Product Inhibition and Further Oxidation

To determine if the slower reaction rate after 40-60 min of incubation was due to enzymatic or non-enzymatic oxidation of propylene oxide or due to product inhibition, 5 $\mu$moles of propylene oxide was added to viable and to heat-killed cell suspensions in the presence and absence of propylene, and incubated at $30^{\circ}C$. Some disappearance in propylene oxide was observed using the viable cell suspension but the amount of propylene

oxide remained constant in the heat-killed cell suspensions, indicating that a slow enzymatic oxidation of propylene oxide was occurring.

Viable cells incubated with both propylene and added propylene oxide showed continued production of propylene oxide; however, the rate of epoxidation was slower than in the standard assay system in which no external propylene oxide was added. Therefore, the further oxidation of propylene oxide, as well as product inhibition, accounted for the apparently slower reaction rate after 60 minutes of incubation.

## pH

The effect of pH on the production of propylene oxide was studied at 30°C using a sodium phosphate buffer (0.05 M) for pH values of from 5 to 7 and a tris(hydroxymethyl)aminomethane buffer (0.05M) for pH values of 8 to 9. The rate of epoxidation of propylene was found not to vary markedly in the pH range from 5 to 9, with the optimum pH about 7. A 5 $\mu$ mole sample of propylene oxide was added to heat-killed cell suspensions at pH 5, 7 and 9 to test for non-enzymatic degradation of propylene oxide at these pH values. The propylene oxide concentration in these suspensions did not decrease during one hour of incubation.

## Temperature

The optimum temperature for the production of propylene oxide by cell suspensions was about 35°C.

## Cell Concentration

The cell concentration also had an influence on the rate of propylene oxide formation. The rate of

production in $\mu$ moles/ml at 30°C increased linearly with increasing cell concentration up to about 16 mg. protein.

## Effect of Propane

The effect of propane on the oxidation of propylene to propylene oxide was studied to determine if a single enzyme system was responsible for the oxidation of both propane and propylene. As shown in Table IX, the addition of propane resulted in reducing the amount of propylene oxide formed.

TABLE IX

Effect of Propane on Production of Propylene Oxide by *Arthrobacter* sp. (CRL 60 P1) NRRL B-11,315)

| Substrate | Propylene Oxide Produced[a] ($\mu$ moles/hr/mg of protein) |
|---|---|
| Propylene | 1.2 |
| Propylene + Propane (1:1, v/v) | 0.82 |

(a) Reactions were carried out as described in Example 1. The product of the reaction was estimated by gas chromatography after 30 minutes of incubation of the reaction mixture at 30°C on a rotary shaker.

## EXAMPLE 9

Optimal Conditions for Oxidation of Alkanes and Secondary Alcohols

The following summarizes tests conducted on the optimal conditions for the production of acetone from propane and 2-propanol and of 2-butanone from butane and 2-butanol using cell suspensions of propane-grown *Arthrobacter* sp. (CRL 60 P1) NRRL B-11,315 as the

representative microorganism strain. It will be understood that these are optimal conditions found for one particular strain and one particular substrate, and the invention is not meant to be bound by them. Conversions of propane, butane, 2-propanol or 2-butanol can still be obtained using *Arthrobacter* sp. (CRL 60 P1) NRRL B-11,315 by deviating from the optimum indicated below, but with lower yields and conversions.

## Time Course

The oxidation of propane and n-butane to acetone and 2-butanone, respectively, by cell suspensions of propane-grown *Arthrobacter* sp. (CRL 60 P1) NRRL B-11,315 during the first 60 minutes of the reaction was linear. On further incubation of the reaction mixture, the rate of production decreased.

The oxidation of 2-propanol and 2-butanol to acetone and 2-butanone, respectively, was linear during the first 120 minutes of incubation. The reactions were carried out in 50mM potassium phosphate buffer of pH 7.0 at $30^{\circ}C$ on a rotary water bath shaker. The production of acetone and 2-butanone was measured at 60 min. whenever the effect of a variable was tested.

Heat-killed cell suspensions of the *Arthrobacter* sp. strain did not catalyze the oxidation of n-alkanes or secondary alcohols.

## pH

The optimum pH for the production of 2-butanone from oxidation of n-butane and 2-butanol was found to be about pH 7.0 and pH 8.0, respectively.

## Temperature

The optimum temperature for the production of 2-butanone from oxidation of n-butane and 2-butanol was found to be about 35°C in both cases. Upon increasing the temperature to 40°C, the rate of production decreased.

## Cell Concentration

The rate of production of acetone from oxidation of propane or 2-propanol and the production rate of 2-butanone from oxidation of butane or 2-butanol was linear with cell protein concentration from 1-12 mg/ml of cell protein. The reactions were carried out in 50mM potassium phosphate buffer of pH 7.0 at 30°C.

## EXAMPLE 10

### Cell-Free Particulate P(40) Fraction:

The Oxidation of Alkenes and Alkanes

Propane-grown *Arthrobacter* sp. (CRL 60 P1) NRRL B-11,315 was grown at 30°C in separate 2.8 liter flasks each containing 700 ml of mineral salts medium as described by J. W. Foster et al., *J. Bacteriol.*, 91, 1924 (1966), *supra* in the presence of propane (propane and air, 1:1 parts by volume) as the sole carbon and energy source. Cells were harvested during exponential growth by centrifugation at 12,000 x g for 15 min. at 4°C. Cells were washed twice with a 25 mM potassium phosphate buffer of pH 7.0 containing 5mM $MgCl_2$ and were then suspended in the same buffer. The resulting cell suspensions at 4°C were disintegrated by a single passage through a French pressure cell (45 mPa) and centrifuged at 5,000 x g for 15 min to remove unbroken

bacteria. The supernatant solution (crude extract) was then centrifuged at 40,000 x g for 60 minutes, yielding particulate P(40) and soluble S(40) fractions. The particulate fraction [P(40)] was suspended in the same buffer as described above and homogenized at 4°C.

A 1.0 ml sample containing the particulate or soluble fraction, 10 μ moles NADH and 150 mM potassium phosphate buffer at pH 7.0 containing 3 μ moles $MgCl_2$ was put into separate 10 ml vials at 4°C which were sealed with a rubber cap. The gaseous phase of the vials was removed by vacuum and replaced with a gas mixture of the indicated alkane or alkene as substrate and oxygen at a 1:1 v/v ratio. The vials were then incubated at 30°C on a rotary shaker at 200 rpm for 10 minutes.

The procedure described in Example 1 (using 2 μl rather than 3 μl samples) was used to evaluate the oxidation products and to determine the oxidation rate of the substrate indicated in Table X using both the particulate and soluble fractions, in the presence of oxygen and NADH, which were required for reaction. The soluble fraction S(40) contained no activity.

Reactions were linear during the first 15 minutes of incubation as measured by detection of product by gas chromatography. The rate of production of epoxide and alcohols by the particulate P(40) fractions is shown in Table X. Both primary and secondary alcohols were identified as oxidation products of the alkanes tested.

TABLE X

Oxidation of Alkenes and Alkanes by Cell-Free Particulate Fraction of *Arthrobacter* sp. (CRL 60 P1) NRRL B-11,315

| Substrate | Product | Rate of Product Formation (μ moles/hr/mg protein) |
|---|---|---|
| Ethylene | ethylene oxide | 0.153 |
| Propylene | propylene oxide | 0.360 |
| 1-Butane | epoxybutane | 0.216 |
| 1-Pentene | epoxypentane | 0.195 |
| Propane | n-propanol | 0.600 |
| | 2-propanol | 0.300 |
| Butane | n-butanol | 0.315 |
| | 2-butanol | 0.150 |

The effect of potential inhibitors on alkane oxidation was investigated by incubating the inhibitors given in Table XI at 1mM concentration with the particulate P(40) fraction of *Arthrobacter* sp. for 15 min. at 0°C before reaction with propane or butane, conducted under the conditions described above. The results are indicated as follows:

TABLE XI

Effect of Inhibitors on Oxidation of Propane and Butane by Cell-Free Particulate P(40) Fraction of *Arthrobacter* sp. (CRL 60 P1) NRRL B-11,315

| Inhibitors | Inhibition (%) |
|---|---|
| 1,10-phenanthroline | 95 |
| Imidazole | 80 |
| Potassium cyanide | 70 |
| Hydroxylamide | 68 |
| α,α-Bipyridyl | 32 |

EXAMPLE 11

Cell-Free Soluble Fraction (secondary alcohol dehydrogenase):

The Oxidation of Secondary Alcohols

Cell suspensions of the microorganism strains identified in Table XII grown on the indicated growth substrate were disrupted with a wave energy ultrasonic oscillator Model W201 (Wave Energy System, Inc., Newton, Pa) and centrifuged at 30,000 x g for 30 min. The supernatant solution (cell extracts) was used for the oxidation of secondary alcohols. Secondary alcohol dehydrogenase activity was measured spectrophotometrically at 340 nm with oxidized nicotinamide adenine dinucleotide ($NAD^+$) as an electron acceptor.

The initial reaction mixture, in a total volume of 3.0 ml, consisted of 50mM phosphate buffer of pH 8.0, 5 μmoles of $NAD^+$, and cell extract. Reaction was initiated by addition of 100 μl of 0.1M 2-butanol as substrate, and the rate of $NAD^+$ reduction was measured and is indicated in Table XII. Protein concentration was determined by the method of Lowry et al., supra (see Example 1).

## TABLE XII

### Oxidation of 2-Butanol by Soluble Extracts of Microorganism Strains

| Microorganism Strain Identification | Growth Substrate | Specific Activity ($\mu$moles of $NAD^+$/min/mg protein reduced) |
|---|---|---|
| Arthrobacter sp. | | |
| (CRL 60 P1) NRRL B-11,315 | ethanol | 0.022 |
| (CRL 60 P1) NRRL B-11,315 | propanol | 0.024 |
| Arthrobacter sp. | | |
| (CRL 60 P1) NRRL B-11,315 | butanol | 0.019 |
| (CRL 60 P1) NRRL B-11,315 | propane | 0.025 |
| (CRL 60 P1) NRRL B-11,315 | propylamine | 0.020 |
| Corynebacterium sp. | | |
| (CRL 63 P4) NRRL B-11,321 | propanol | 0.018 |
| Mycobacterium sp. | | |
| (CRL 62 P3) NRRL B-11,323 | propanol | 0.024 |
| Nocardia sp. | | |
| (CRL 64 P5) NRRL 11,327 | propanol | 0.016 |
| Pseudomonas sp. | | |
| (CRL 65 P6) NRRL B-11,332 | propanol | 0.020 |

Soluble extracts of Arthrobacter sp. (CRL 60 P1) NRRL B-11,315 were used to study the oxidation of the secondary alcohols given in Table XIII using the procedure described above.

TABLE XIII

Oxidation of Secondary Alcohols by Soluble Extracts of *Arthrobacter* sp. (CRL 60 P1) NRRL B-11,315

| Substrate | Rate of Oxidation (%)[a] |
|---|---|
| Isopropanol | 65 |
| 2-Butanol | 100 |
| 2-Pentanol | 12 |
| 2-Hexanol | 10 |
| 2-Heptanol | 4 |

(a) A 100% rate of oxidation was 22 nmoles of $NAD^+$ reduced per minute per mg of protein as determined by spectrophotometric assay.

Soluble extracts of *Arthrobacter* sp. (CRL 60 P1) NRRL B-11,315 were used to study the inhibitory effect of metal-binding agents and sulfhydryl inhibitors on the oxidation of 2-butanol. All inhibitors were tested at 1mM concentration and incubated with the soluble extracts in the reaction mixture for 15 min at $0^oC$. Otherwise the procedure described above was followed. The results are indicated in Table XIV.

TABLE XIV

Effect of Inhibitors on Oxidation of 2-Butanol by Cell Extracts of *Arthrobacter* sp. (CRL 60 P1) NRRL B-11,315

| Inhibitors | Inhibition (%)[(a)] |
|---|---|
| α,α-Bipyridyl | 30 |
| 1,10-Phenanthroline | 80 |
| Potassium cyanide | 20 |
| Imidazole | 90 |
| Cupric sulfate | 100 |
| p-Hydroxymercuribenzoate | 95 |
| N-Ethylmaleimide | 30 |

(a) The uninhibited activity of 2-butanol was 25 nmoles of $NAD^+$ reduced per min per mg of protein.

The optimum pH for the oxidation of 2-butanol by the soluble extracts of *Arthrobacter* sp. (CRL 60 P1) NRRL B-11,315 in the presence of $NAD^+$ as an electron acceptor was found to be 8.5.

EXAMPLE 12

Cell-Free Soluble Fraction (alkane monooxygenase):

The Epoxidation of Alkenes and Styrene

The microorganism *Brevibacterium* sp. (CRL 56 P6Y) NRRL B-11,319 was grown on propane (7% propane and 93% air) at 30°C in a batch culture on a mineral salt medium as described in Example 1 in a 30-liter fermentor (New Brunswick Scientific Co., Edison, N.J.). The fermentor was inoculated with 2 liters of a culture grown in flasks.

The cells thus grown were washed twice with 25 mM potassium phosphate buffer pH 7.0 and suspended in the same buffer solution containing 5mM $MgCl_2$ and deoxyribonuclease (0.05 mg/ml). Cell suspensions at $4^oC$ were disintegrated by a single passage through a French pressure cell (American Instruments Co., Silver Spring, Md.) at 60 mPa. Disintegrated cell suspensions were centrifuged at 15,000 x g for 15 min. to remove unbroken cells. The supernatant solution was then centrifuged at 40,000 x g for 60 minutes and the supernatant solution therefrom was again centrifuged at 80,000 x g for 60 minutes, yielding the soluble fraction.

Several 3-ml vials at $4^oC$ were charged with 0.2 ml of a reaction mixture consisting of 10 $\mu$ moles potassium phosphate buffer pH 7.0, 4 $\mu$ moles $NADH_2$, and the soluble enzyme fraction obtained above. The gaseous phase of the vials was evacuated by vacuum and replaced with a gas mixture of gaseous oxidation substrate and oxygen (1:1 v/v); in the case of a liquid oxidation substrate, 2 $\mu$l of substrate was added. The vials were incubated at $35^oC$ on a reciprocating water bath shaker at 50 oscillations per minute.

The rate of epoxidation of alkenes and styrene was measured by injecting 1-2 $\mu$l samples of the reaction mixture into a gas chromatograph immediately after addition of substrate (zero time) and after 5 and 10 min. of incubation. Specific activities were expressed as $\mu$ moles of product formed per 10 min. per mg of protein. With each substrate, control experiments were conducted in the absence of $NADH_2$, in the absence of oxygen, and using boiled extracts. The results obtained are shown in Table XV.

TABLE XV

Epoxidation of Alkenes and Styrene by Soluble Extracts of *Brevibacterium* sp. (CRL 56 P6Y) NRRL B-11,319

| Oxidation Substrate | Product | Rate of Product Formation ($\mu$ mole/10 min./mg protein) |
|---|---|---|
| Ethylene | Ethylene oxide | 0.040 |
| Propylene | Propylene oxide | 0.052 |
| 1-Butene | 1,2-Epoxybutane | 0.030 |
| 1,3-Butadiene | 1,2-Epoxybutene | 0.044 |
| Isobutene | Epoxyisobutane | 0.046 |
| *cis*-But-2-ene | *cis*-2,3-Epoxybutane | 0.018 |
| *trans*-But-2-ene | *trans*-2,3-Epoxybutane | 0.022 |
| 1-Pentene | 1,2-Epoxypentane | 0.025 |
| 1-Hexene | 1,2-Epoxyhexane | 0.019 |
| 1-Heptene | 1,2-Epoxyheptane | 0.008 |
| Styrene | Styrene oxide | 0.005 |

The Oxidation of Alkanes, and Cyclic and Aromatic Compounds

The procedure described above was employed using the oxidation substrates given in Table XVI. The results are provided in the table.

TABLE XVI

Oxidation of Alkanes, Cyclohexane and Toluene by Soluble Extracts of *Brevibacterium* sp. (CRL 56 P6Y) NRRL B-11,319

| Oxidation Substrate | Product | Rate of Product Formation (μmole/10 min./mg protein) |
|---|---|---|
| Ethane | Ethanol | 0.0090 |
| Propane | 1-Propanol | 0.0054 |
| | 2-Propanol | 0.0060 |
| Butane | 1-Butanol | 0.0062 |
| | 2-Butanol | 0.0065 |
| Isobutane | Isobutanol | 0.0044 |
| Pentane | 1-Pentanol | 0.0035 |
| | 2-Pentanol | 0.0043 |
| Hexane | 1-Hexanol | 0.0048 |
| | 2-Hexanol | 0.0032 |
| Cyclohexane | Cyclohexanol | 0.0065 |
| Toluene | Benzyl alcohol | 0.0020 |

EXAMPLE 13

Regeneration of Electron Carrier

It has been found that the monooxygenase which catalyzes the epoxidation reaction requires an electron carrier (cofactor) such as NADH or NADPH for its activity. When the cofactor is depleted, it can be regenerated by the addition of compounds which are substrates for dehydrogenases or oxidases such as alcohol (primary and/or secondary) dehydrogenase, aldehyde dehydrogenase, formate dehydrogenase, steroid dehydrogenase, etc. in either cell-free or whole cell systems. The overall catalyst system is stabilized by coupling this cofactor regeneration system to the epoxidation reaction process.

A schematic explanation of the coupling cycle for the regeneration of cofactor is shown below:

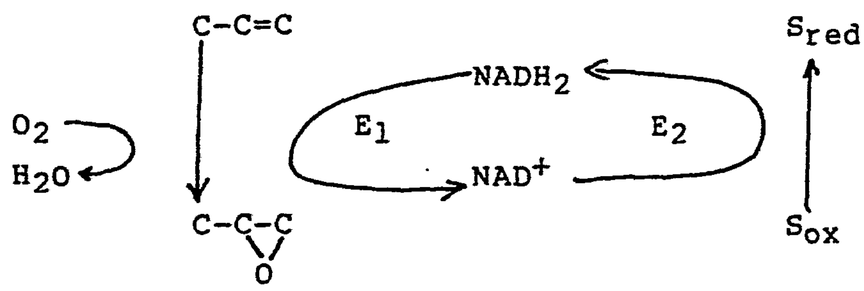


wherein $E_1$ is the monooxygenase enzyme, $E_2$ is the dehydrogenase enzyme and S is the substrate for cofactor regeneration.

<u>Cofactor regeneration through ethanol dehydrogenase</u>

<u>Arthrobacter</u> sp. (CRL 60 P1) NRRL B-11,315 was grown on propane and used to epoxidize propylene to propylene oxide as described in Example 1, except that varying amounts of ethanol were added to the reaction mixture in separate experiments, the ethanol being a cofactor regeneration substrate. Ethanol was converted to acetaldehyde (acetyl Co ~ A in vivo) by primary alcohol dehydrogenase, yielding 1 mole of NADH, with subsequent oxidation of the intermediate acetyl Co ~ A to carbon dioxide, yielding 3 additional moles of NADH. The results, indicated in Table XVII, show that the epoxidation rate was improved on addition of 5 $\mu$ mole ethanol. Some inhibition was observed at higher ethanol concentration (25 $\mu$ moles) within the first two hours of reaction.

TABLE XVII

| Amount of ethanol | Epoxidation Rate (rate of propylene oxide formation in μmole/0.5 ml.) 1 hr. | 2 hr. | 3 hr. |
|---|---|---|---|
| 0 (Control) | 2.5 | 3.8 | 3.9 |
| 5 μmole | 2.9 | 5.6 | 6.5 |
| 25 μmole | 2.4 | 5.2 | 6.5 |

In summary, the present invention is seen to provide newly discovered and isolated microorganism strains and their genetically engineered derivatives which are capable of growth under aerobic conditions in a culture medium containing a $C_2$-$C_{10}$ alkyl compound. Such microorganisms are particularly useful in the oxidation of oxidizable organic substrates under aerobic conditions, more particularly, saturated hydrocarbons to alcohols and/or ketones, olefins to epoxides, and secondary alcohols to methyl ketones.

CLAIMS:

1. Isolated and biologically pure microbial cultures of microorganisms which utilize $C_2$-$C_{10}$ alkyl compounds, said cultures being selected from the group having the following identifying characteristics:

| | | |
|---|---|---|
| Acinetobacter sp. | (CRL 67 P11) | NRRL B-11,313 |
| Actinomyces sp. | (CRL 66 P7) | NRRL 11,314 |
| Arthrobacter sp. | (CRL 60 P1) | NRRL B-11,315 |
| Arthrobacter sp. | (CRL 68 E1) | NRRL B-11,316 |
| Arthrobacter sp. | (CRL 70 B1) | NRRL B-11,317 |
| Brevibacterium sp. | (CRL 52 P3P) | NRRL B-11,318 |
| Brevibacterium sp. | (CRL 56 P6Y) | NRRL B-11,319 |
| Brevibacterium sp. | (CRL 61 P2) | NRRL B-11,320 |
| Corynebacterium sp. | (CRL 63 P4) | NRRL B-11,321 |
| Mycobacterium sp. | (CRL 51 P2Y) | NRRL B-11,322 |
| Mycobacterium sp. | (CRL 62 P3) | NRRL B-11,323 |
| Mycobacterium sp. | (CRL 69 E2) | NRRL B-11,324 |
| Nocardia sp. | (CRL 55 P5Y) | NRRL 11,325 |
| Nocardia sp. | (CRL 57 P7Y) | NRRL 11,326 |
| Nocardia sp. | (CRL 64 P5) | NRRL 11,327 |
| Pseudomonas sp. | (CRL 53 P3Y) | NRRL B-11,329 |
| Pseudomonas sp. | (CRL 54 P4Y) | NRRL B-11,330 |
| Pseudomonas sp. | (CRL 58 P9Y) | NRRL B-11,331 |
| Pseudomonas sp. | (CRL 65 P6) | NRRL B-11,332 |
| Pseudomonas sp. | (CRL 71 B2) | NRRL B-11,333 |

and genetically engineered derivatives and natural mutants thereof, said cultures being characterized as capable of reproducing themselves and capable of producing dehydrogenase and/or monooxygenase enzyme activity in isolatable amounts when cultured under aerobic conditions in a liquid growth medium comprising assimilable sources of nitrogen and essential mineral salts in

the presence of a $C_2$-$C_{10}$ alkyl compound as the major carbon and energy source.

2. A culture according to claim 1 in which the $C_2$-$C_{10}$ alkyl compound is an alkane, alcohol or alkylamine.

3. A culture according to claim 1 in which the $C_2$-$C_{10}$ alkyl compound is a $C_2$-$C_4$ n-alkane, a $C_2$-$C_4$ primary alcohol or a $C_2$-$C_4$ alkylamine.

4. A process for the production of microbial cells which comprises culturing, under aerobic conditions, in a liquid growth medium containing assimilable sources of nitrogen and essential mineral salts in the presence of a $C_2$-$C_{10}$ alkyl compound, any one of the microorganism strains defined in claim 1.

5. A process according to claim 4 in which the culturing takes place at a temperature ranging from about 5 to about 50°C and at a pH in the range from about 4 to 9.

6. A Process for advancing an oxidizable organic substrate containing at least two carbon atoms to a state of oxidation greater than its original state, characterized by contacting the substrate, in a reaction medium under aerobic conditions, with cells of a microorganism defined in claim 1, a genetically engineered derivative or natural mutant thereof, or an enzyme preparation prepared from the cells or derivative, which microorganism, derivative, mutant or preparation exhibits oxygenase and/or hydrogenase enzyme activity, until at least a portion of the corresponding oxidation product is produced in isolatable amounts, wherein the microorganism has been aerobically cultivated in a nutrient medium containing a $C_2$-$C_{10}$ alkyl compound.

7. A process according to claim 6 characterized in that the enzyme preparation is derived from cell-free extracts of the microorganism.

8. A process according to claim 7 characterized in that an electron carrier is added to the reaction medium.

9. A process according to any one of claims 6 to 8 in which the substrate is an alkane, cycloaliphatic or aromatic hydrocarbon, alkene, diene, or vinyl aromatic compound, and the $C_2$-$C_{10}$ alkyl compound is an alkane.

10. A process according to any one of claims 6 to 8 in which the substrate is a secondary alcohol and the $C_2$-$C_{10}$ alkyl compound is a $C_2$-$C_4$ alkyl compound.